Europäisches Patentamt

European Patent Office

Office européen des brevets

(19)

(11) Publication number: **0 459 577 A2**

# (12) EUROPEAN PATENT APPLICATION

(21) Application number: 91201243.2

(22) Date of filing: 25.05.91

(51) Int. Cl.⁵: **C07K 15/28**, A61K 39/395, C12N 15/13

(30) Priority: 01.06.90 US 532001

(43) Date of publication of application:
04.12.91 Bulletin 91/49

(84) Designated Contracting States:
AT BE CH DE DK ES FR GB IT LI LU NL SE

(71) Applicant: MERCK & CO. INC.
126, East Lincoln Avenue P.O. Box 2000
Rahway New Jersey 07065-0900(US)

(72) Inventor: Colonno, Richard J.
17 Brookdale Drive
New Britain Township, PA 18901(US)
Inventor: Condra, Jon H.
1824 Silver Avenue
Abington, PA 19001(US)
Inventor: Tomassini, Joanne E.
2058, Spring Valley Road
Lansdale, PA 19446(US)
Inventor: Sardana, Vinod V.
945, Flintlock Drive
Lansdale, PA 19446(US)

(74) Representative: Barrett-Major, Julie Diane et al
Merck & Co., Inc. European Patent
Department Terlings Park Eastwick Road
Harlow Essex CM20 2QR(GB)

(54) Microbially expressed portions of a monoclonal antibody block rhinovirus attachment to cell receptors.

(57) Complementary DNA (cDNA) fragments encoding the Fab and Fv portion of the light and heavy chains of a murine monoclonal antibody (mAb) were prepared from hybridoma messenger RNA (mRNA). The mAb is specific for the intercellular adhesion molecule-1 (ICAM-1). Furthermore, the mAb blocks the attachment of human rhinovirus (HRV) particles to their cellular receptor, ICAM-1. Fab and Fv cDNA were molecularly cloned and expressed in a recombinant host, purified, and shown to be biologically active in that they bind to ICAM-1, and block attachment of ligands to ICAM-1, including HRV.

FIG. II

EP 0 459 577 A2

## BACKGROUND OF THE INVENTION

Using an antibody known to prevent the attachment of the major group of HRV, and immunoaffinity chromatography, a 90 kilodalton (kDa) glycoprotein was isolated from HeLa cell membranes and was subsequently shown to be the receptor protein required for the attachment of the major group of HRV to susceptible cells. Biochemical characterization of the purified 90 kDa receptor protein determined that it was an acidic glycoprotein and contained seven N-linked glycosylation sites. Recent cDNA cloning experiments have identified the receptor as the intercellular adhesion molecule-1 (ICAM-1). ICAM-1 is a cell surface ligand for the lymphocyte function-associated antigen-1 adhesion receptor (LFA-1) and is a member of the immunoglobulin supergene family, since it is predicted to contain five homologous immunoglobulinlike domains. Some sequence homology exists between ICAM-1 and other adhesion proteins of the adult nervous system, namely, neural cell adhesion molecule and myelin-associated glycoprotein. The interaction between ICAM-1 and LFA-1 plays an important role in leukocyte adhesion and in the execution of immunological and inflammatory functions mediated by leukocyte adhesion. LFA-1 is a member of the CD18 family of lymphocyte adhesion proteins. As a result of this adhesion, lymphocytes are capable of important and beneficial immunologic functions, including surveilance for foreign antigens. However, this same adhesion activity can also lead to non-desirable effects such as transplant rejection, other autoimmune disease states, and complications resulting from inflammation.

ICAM-1 is a glycoprotein present on the surface of a wide variety of cell types including vascular endothelial cells, thymic epithelial cells as well as certain other epithelial and fibroblastic cells, macrophages, T-lymphocyte blasts, germinal center B cells, and dendritic cells of lymph nodes. ICAM-1 is preferentially expressed at sites of inflammation and not in resting or quiescent cells. High levels of ICAM-1 expression can be induced in dermal fibroblasts upon exposure to cytokines, such as gamma interferon, or interleukin-1.

The major HRV group receptor is a 90 kDa glycoprotein that was recently cloned and identified as ICAM-1 [Greve, J.M. et al. (1989), Cell, 56, pp. 839-847, and Tomassini J. et al., (1989), PNAS, 86, pp. 4907-4911]. The cellular receptor for the minor group is more elusive and has been tentatively identified as a 120 kDa protein.

Using HeLa cell membranes as an immunogen, a mouse IgG-1 mAb, designated 1A6, was isolated that specifically blocked the major group of HRV from binding to and infecting HeLa cells [Colonno, R.J. et at., (1986), J. Virol., 57, pp. 7-12]. In addition, 1A6 and its proteolytically generated Fab fragment were capable of displacing cell-bound HRV virions, in vitro, and showed no evidence of cytotoxicity to dividing cells despite prolonged exposure. These studies also demonstrated that virus access to susceptible cells could only be achieved through interaction with the ICAM-1 receptor, since challenge with high virus titer inocula failed to bypass the receptor blockade caused by 1A6. These results suggested that 1A6 may be useful as an antiviral agent in the prevention or treatment of common colds in humans. This notion was supported by studies showing that intranasally-administered 1A6 was effective in blocking HRV infection of chimps, and in modulating the clinical symptoms of HRV infection in human clinical trials [Hayden, F.G., (1988), Antiviral Res., 9, pp. 233-247].

ICAM-1 may be involved in other diseases including asthma, chronic bronchitis, emphysema, and idiopathic pulmonary fibrosis. ICAM-1 may also contribute to the onset and progression of other diseases chracterized by eosinophil infiltration and tissue sensitization, including rhinitis, nasal polyposis, chronic urticaria, and atopic dermatitis.

## OBJECTS OF THE INVENTION

It is an object of the present invention to provide portions of antibody molecules, produced in a recombinant host cell, which preserve the antigen recognition of the natural antibody. It is an additional object of the present invention to provide portions of antibody molecules, produced in a recombinant host cell, specific for the intercellular adhesion molecule-1 (ICAM-1). Another object of the present invention is to provide portions of antibody molecules which can prevent attachment of the major HRV group to, or displace attached HRV from, their cellular receptor, ICAM-1. It is also an object of the present invention to provide portions of antibody molecules produced in a recombinant host cell, which can bind to ICAM-1 and inhibit the biological activity and function of ICAM-1 as an intercellular adhesion molecule. A further object of the present invention is to provide pharmaceutical formulations for administration to humans, containing a portion or portions of antibodies produced in a recombinant host cell, which can prevent attachment of the major HRV group to, or displace attached HRV from, their cellular receptor, ICAM-1. An additional object of the present invention is to provide pharmaceutical formulations for administration to humans, containing a

2

portion or portions of an antibody molecule, produced in a recombinant host cell, which can bind to ICAM-1 and inhibit the biological activity and function of ICAM-1 as an intercellular adhesion molecule. These and other objects of the present invention will be apparent from the following description.

SUMMARY OF THE INVENTION

Complementary DNA (cDNA) fragments were cloned which encode the antigen recognition region of mAb's specific for ICAM-1. These cDNA's were expressed in a recombinant host cell, purified, and were shown to possess the biological properties of antibody-antigen interaction of the natural antibodies from which they were derived. Specifically, the recombinant antibody portions bind to ICAM-1 and can prevent attachment of the major HRV group to their cellular receptor, ICAM-1. In addition, the recombinant antibody portions displace attached HRV from ICAM-1. These recombinant antibody fragments are useful in pharmaceutical formulations for the prevention or amelioration of many human diseases in which ICAM-1 is involved, including inflamation and other autoimmune diseases, and HRV infection.

BRIEF DESCRIPTION OF THE DRAWINGS

Figure I - A schematic diagram of: A - an intact immunoglobulin molecule (IgG); B - Fab portion of an immunoglobulin molecule; C - single chain Fv. The dotted line in C represents the peptide bridge joining the H and L chains.

Figure II - Antibody-mediated inhibition of HRV attachment to susceptible cells. Confluent monolayers of HeLa cells in 48 well plates were treated in duplicate with the indicated amounts of mAb 1A6, 2C2, or 18B9 for 30 minutes at 34°C. $^{35}$S-labelled HRV-36 was added, and the cells were incubated for 1 hour. The amount of unbound HRV and bound HRV was measured and percent inhibition was determined.

Figure III - A - Plasmid pG1, arrows indicate the direction of transcription; B - detail of the junctions of H chain cDNA in the plasmid, and the corresponding amino acid sequence with Kabat numbering designations.

Figure IV - A - Plasmid pK1, arrows indicate the direction of transcription; B - detail of the junctions of L chain cDNA in the plasmid, and the corresponding amino acid sequence with Kabat numbering designations.

Figure V - A - Plasmid pSCV1, arrows indicate the direction of transcription; B - detail of the junctions of H and L chain cDNA in the plasmid, and the corresponding amino acid sequence with Kabat numbering designations of both H and L chains.

Figure VI - HRV receptor blockage by recombinant antibody fragments. HeLa cell membranes were incubated with various amounts of the antibody, or recombinant antibody fragments for 20 minutes, followed by the addition of $^{35}$S-labelled HRV-15 and incubation for one hour. The membranes were separated by centrifugation and the amount of $^{35}$S-labelled HRV-15 adsorbed to the membranes was measured and indicated as percent reduction of virus binding. ● - 1A6 antibody, ▽ - natural Fab, □ -recombinant Fab, △ - Fab(-SS), ▲ - pSCV1, ○ -pSCV2, ■ - pSCV3.

Figure VII - Cell-free translation and immunoprecipitation of ICAM-1 domain 1. In vitro-transcribed RNA resulting from the polymerase chain reaction-amplified modification of the domain 1 fragment was translated in the presence of added microsomal membranes and then spiked with an in vitro translated yeast alpha mating protein (Promega Biotec) prior to immunoprecipitation and SDS-PAGE analysis. The control lane shows the position of yeast alpha mating protein alone, while lane 1 shows the control protein and ICAM-1 domain-1 together. The material generated in lane 1 was utilized in immunoprecipitations with MAb's OKT4 (lane 2), 1A6 (lane 3), 2C2 (lane 4), and 18B9 (lane 5). The positions of low molecular weight markers (KD) are displayed in lane M.

DETAILED DESCRIPTION OF THE INVENTION

ICAM-1 is a cell surface glycoprotein which functions as a ligand for lymphocyte function-associated antigen-1 receptor (LFA-1). ICAM-1 is also the receptor for the attachment of the major group of HRV to susceptible cells. ICAM-1 is a member of the immunoglobulin supergene family since it is predicted to contain five homologous immunoglobulin-like domains [Simmons, D. et al., (1988) Nature, 331, pp 624-27]. The interaction of ICAM-1 and LFA-1 plays an important role in leukocyte adhesion, and in the execution of immunological and inflammatory functions mediated by leukocyte adhesion. LFA-1 is a member of the CD18 family of lymphocyte adhesion proteins.

Antibodies which bind to the CD18 lymphocyte adhesion proteins have been shown to inhibit, in vitro,

3

lymphocyte functions which are dependent upon adhesion, including the attachment of granulocytes to endothelial cells [Smith, C.W. et at., (1988), J. Clin. Invest., 82, pp. 1746-56].

Furthermore, individuals suffering from leukocyte adherence deficiency disease, an inherited genetic defect, do not produce CD18 related proteins. As a result, granulocytes from these individuals exhibit adhesion incapacity as observed with normal granulocytes in the presence of anti-CD18 antibodies described above.

CD18, therefore, enables leukocytes to adhere to various cells including other leukocytes, vascular endothelial cells, and other non-vascular cells. The ligand to which CD18 proteins bind for adhesion is ICAM-1.

Inflammation can result from the reaction and response of the host's immunologic system to specific antigens. Certain autoimmune diseases and T-cell mediated delayed-type hypersensitivity are examples of this type of inflammation. Leukocyte adhesion is also involoved in other disease states including transplant rejection, and tissue graft rejection. Some of the desirable immunological functions resulting from leukocyte adhesion include antibody production, and destruction of virus infected cells.

ICAM-1 is therefore a cellular substrate to which lymphocytes can attach in order to perform various functions. Since ICAM-1 molecules are expressed at high levels at sites of inflammation, including delayed-type hypersensitivity, interruption of cellular adhesion mediated by ICAM-1 can modify inflammatory responses.

Antibodies, or recombinantly expressed portions of antibody molecules, have significant therapeutic value in the diminution or elimination of the inflamatory responses, provided that the antibody, or recombinantly expressed portion of an antibody molecule, can bind to ICAM-1. An antibody, or recombinantly produced portion thereof, can be administered to an individual prior to experiencing, or during, an inflammatory response and alleviate the consequences.

The protein domains of mAb's that confer the antigen recognition determinants can be separated from the remainder of the antibody molecule, and retain their antigen binding ability. The Fab portion of an antibody (e.g., IgG) is approximately one third (about 55 Kilo Daltons) of the intact antibody, and exhibits monovalent antigen binding capabilities (See Figure I).The structure of Fab is much simpler than an intact immunoglobulin molecule; Fab having only 5 of 16 disulfide bonds found in an intact IgG molecule (See Figure I ). The Fab portion carries the antigen binding site and is composed of two separate polypeptide chains, the heavy (H) chain variable domain, with the first constant domain and part of the hinge region, and the entire light (L) chain. The two chains (H and L) are covalently linked by a single disulfide bond between the constant domains of each chain. Fab can be further dissected to yield a smaller (about 25 KD) molecule capable of monovalent antigen binding, termed Fv (See Figure I). Fv carries the antigen binding site, and conists only of non-covalently associated H and L chain variable domains. The antigen affinity of the entire Fv portion is identical to the corresponding Fab which, in turn, is identical to its corresponding immunoglobulin molecule.

Both Fab and single chain Fv portions have been expressed in recombinant hosts cells, including mammalian cell cultures and E. coli. Fab and Fv are advantageous over intact antibodies for therapeutic and diagnostic use, in that their smaller size permits more efficient penetration of tissue boundaries. Furthermore, amenability to expression in recombinant hosts such as E. coli, greatly facilitates scale up to high level production of Fab and Fv. In addition, recombinant DNA techniques can be used to alter the structure of the antibody portion. An example of such alteration would be the exchange of mouse constant regions of a mouse antibody molecule, for a human constant region, forming a "humanized" antibody or antibody portion. The human constant region is less likely to elicit an immune response from a human subject upon administration of the antibody portions, than would an antibody containing the mouse constant region.

Recombinant DNA techniques can produce further alterations in antibody molecules, or portions thereof, including combinations of light and heavy chain portions from different sources, whether the sources are from different species, different immunoglobulin classes, different antigen specificity, and varying the fusion point between variable and constant regions. Antibodies or portions thereof, can be constructed, for example, which possess higher antigen affinity, or incorporate improvements found in a different constant region.

When these proteins are expressed in E. coli, the H and L chains may require denaturing, renaturing, and mixing at the proper ratios in order to form a biologically active molecule with the appropriate secondary and tertiary structure. To help alleviate these problems, the two components of Fv (the H and L chains) can be recombinantly synthesized in host cells as a fused polypeptide chain. In addition, cysteine residues can be removed by nucleotide substitution to eliminate disulfide bond formation.

In order to generate mAb's that block the binding of HRV to the virus receptor on susceptible cells, mice were immunized with whole human cells, and human cell membranes enriched for HRV receptor sites,

4

e.g., HeLa cells. Hybridomas were produced following fusion of spleen cells from immunized mice with mouse myeloma cells [Colonno et al., J. Virol., 57, pp 7-12, 1986]. Three mAb's, 1A6, 2C2, and 18B9, were selected that were each capable of blocking the attachment of HRV to receptor sites on susceptible cells (Figure II). This analysis was extended to demonstrate the ability of 1A6, 2C2, and 18B9 to block attachment of at least 91 different HRV serotypes to susceptible cells. The specific viral serotypes which were unable to cause infection in the presence of one of the mAb as well as those sterotypes which were able to cause infection in the presence of one of the mAb are shown in Table I.

From these experiments it has been determined that HRV consist of a major group and a minor group. The major group is rendered incapable of causing infection in the presence of the mAb while the minor group is capable of causing infection in the presence of the mAb.

It has also been found that these mAb bind strongly to susceptible cells and possess greater avidity for the cellular receptor than HRV already bound to the cell [Colonno, R.J. et al., (1986), J.Virol., pp. 7-12].

The mAb's as well as recombinantly expressed portions thereof (for example Fab and single chain Fv), are useful for the prevention of HRV infection, or to ameliorate the duration and severity of HRV infection. Treatment of susceptible cells with the mAb or recombinantly expressed portions thereof, including Fab and single chain Fv, render the cells resistant to HRV infection for a period of up to 50 hours following removal of excess antibody.

cDNA encoding ICAM-1 has been cloned and characterized [Tomassini, J.E. et al. , (1989), P.N.A.S. (USA), 86, pp4907 - 11] Fragments of ICAM-1 cDNA were transcribed and translated, in vitro, generating a series of $CO_2$H-terminal truncations. Immunoprecipitation of these ICAM-1 fragments using mAb's 1A6, 2C2, and 18B9 was used to identify the mAb binding sites on ICAM-1.

Experiments utilizing a subset of ICAM-1 polypeptides clearly showed that mAb's 1A6, 2C2, and 18B9 were able to recognize and immunoprecipitate an $NH_2$-terminal ICAM-1 fragment which represents the first 82 amino acids of the mature ICAM-1 molecule (see Figure VII). This portion of ICAM-1 encompasses only domain 1 of the five predicted domains of mature ICAM-1. 1A6, 2C2, and 18B9 are therefore specific for domain 1 of ICAM-1.

A full-length cDNA clone (pHRVr1) was shown to have a single, large, open reading frame initiating at nucleotide 72 that encodes 532 amino acids and contains a 1,333-nucleotide 3' noncoding region. Signal and transmembrane sequences are predicted to exist near the $NH_2$- and $CO_2$H-terminal regions of the molecule, respectively. A full-length subclone was inserted into a pGEM4Z transcription vector to form pGEM-ICAM. The orientation of the cloned sequence is such that the 5'-terminus is just downstream of the phage SP6 promoter. Thus, specific ICAM-1 mRNA can be transcribed from linearized plasmid DNA with SP6 RNA polymerase. Prior to transcription, plasmid DNA was cleaved with conveniently located restriction enzymes to enable generation of RNA fragments encoding selective domains of the ICAM-1 receptor molecule.

In vitro translation of the resulting RNAs in rabbit reticulocyte lysates generated polypeptides of the expected molecular weights as determined by SDS-PAGE. Since ICAM-1 is a membrane protein and has been shown to contain seven N-linked glycosylation sites, canine microsomal membranes were added to the translation mixture to optimize the secondary structure formation of ICAM-1 molecules.

Translation of full-length ICAM-1 mRNA in the presence of microsomal membranes and 2mM oxidized glutathione resulted in two major protein bands with molecular sizes of approximately 55 and 70 kDa. The higher molecular weight band presumably results from glycosylation activity associated with microsomal membranes. Larger protein species were also observed with each of the other ICAM-1 RNA's except the domain 1 fragment which continued to generate a 16-kDa polypeptide. This result was expected, since the domain 1 amino acid sequence is devoid of potential glycosylation sites.

Immunoprecipitation experiments were done which demonstrated that each of the ICAM-1 polypeptide fragments synthesized in the presence of microsomal membranes was specifically precipitated by each of the mAbs (Figure VII). Radiolabelled proteins encoding polypeptides as short as 82 amino acids were still recognized by all three mAbs. Two antibody specificity controls were incorporated into these experiments. The first control involved the parallel testing of mAb OKT4, which recognizes the CD4 receptor; the second control was the addition of radiolabelled globin, or yeast mating factor, to each immunoprecipitation sample. Both of these controls indicated that the immunoprecipitations were specific, since mAb OKT4 was unable to recognize the ICAM-1 polypeptides, and globin was not immunoprecipitated by the anti-ICAM-1 mAbs.

## TABLE I

### CHARACTERIZATION OF MONOCLONAL ANTIBODIES
### TO HRV CELLULAR RECEPTOR

ANTIBODY 1A6, 2C2, or 18B9:

(MAJOR HRV GROUP)
PROTECTION AGAINST INFECTION BY:

| | | | | | | | | | |
|---|---|---|---|---|---|---|---|---|---|
| HRV-3 | HRV-12 | HRV-21 | HRV-33 | HRV-42 | HRV-54 | HRV-64 | HRV-73 | HRV-82 | HRV-92 |
| HRV-4 | HRV-13 | HRV-22 | HRV-34 | HRV-43 | HRV-55 | HRV-65 | HRV-74 | HRV-83 | HRV-93 |
| HRV-5 | HRV-14 | HRV-23 | HRV-35 | HRV-45 | HRV-56 | HRV-66 | HRV-75 | HRV-84 | HRV-94 |
| HRV-6 | HRV-15 | HRV-24 | HRV-36 | HRV-46 | HRV-57 | HRV-67 | HRV-76 | HRV-85 | HRV-95 |
| HRV-7 | HRV-16 | HRV-25 | HRV-37 | HRV-48 | HRV-58 | HRV-68 | HRV-77 | HRV-86 | HRV-96 |
| HRV-8 | HRV-17 | HRV-26 | HRV-38 | HRV-50 | HRV-59 | HRV-69 | HRV-79 | HRV-88 | HRV-97 |
| HRV-9 | HRV-18 | HRV-27 | HRV-39 | HRV-51 | HRV-60 | HRV-70 | HRV-79 | HRV-89 | HRV-98 |
| HRV-10 | HRV-19 | HRV-28 | HRV-40 | HRV-52 | HRV-61 | HRV-71 | HRV-80 | HRV-90 | HRV-99 |
| HRV-11 | HRV-20 | HRV-32 | HRV-41 | HRV-53 | HRV-63 | HRV-72 | HRV-81 | HRV-91 | HRV-100 |

CAV-13    CAV-18    CAV-21                                              HRV-HANKS

(MINOR HRV GROUP)
NO PROTECTION AGAINST INFECTION BY:

| | | | |
|---|---|---|---|
| HRV-1A | HRV-29 | HRV-44 | HRV-62 |
| HRV-1B | HRV-30 | HRV-47 | HRV-87 |
| HRV-2 | HRV-31 | HRV-49 | |

The mAb or recombinantly expressed portions thereof, including Fab and single chain Fv, may be prepared in a suitable topical formulation for administration in the form of drops or as a spray. The recombinant portions of antibody molecules may also be administered in pharmaceutical compositions for injection purposes. With the aid of suitable liquids, the recombinant antibody fragments can be used in injection preparations in the form of liquids. These pharmaceutical compositions comprise a pharmaceutically effective amount of the recombinant protein or proteins, together with a pharmaceutically acceptable carrier. A patient can be treated, for example, parenterally, with an inflammation reducing, effective amount of the recombinant protein to eliminate or at least reduce the degree of inflammation. The dose will, of course, vary in accordance with the factors well known and understood by the physician such as age, weight, and general physical condition of the patient. In general, a dose range between about 1 pg to 10 mg of the antibody portion per kilogram of patient weight may be administered, although a higher or lower dose may be used.

When administered by nasal aerosol inhalation, the formulations may be prepared as solutions in saline, employing benzyl alcohol or other suitable preservatives, absorption promoters to enhance bioavailability, flourocarbons, and/or other solubilizing or dispersing agents known in the art. The dosage may range from about 1 $\mu$g to about 1 mg given at intervals of about 2 hours to about 12 hours. A typical formulation employs the mAb fragments of the present invention in PBS containing a stabilizer, e.g., 20% SPGA.

Full-length cDNA clones of 1A6, 2C2, and 18B9 hybridoma heavy (H) and light (L) chains were isolated and modified for expression in E. coli using the polymerase chain reaction (PCR) technique. For expression of Fab H (termed in the art, Fd) and L chains, a unique NdeI site and an initiator methionine codon were inserted immediately 5' of the sequence encoding the signal peptidase cleavage site of both H and L chains. In the H chain clone, a termination codon followed by a Bg1II site were inserted after amino acid 236 (Kabat numbering system) [Kabat, E. A. etal., (1987), Sequences of Proteins of Immunological Interest, 4th Ed., U. S. Government Printing Office, Washington D. C.], one residue beyond the cysteine normally bridging the H and L chains. For L chain expression, a Bg1II site was introduced 3' of the natural termination codon. Both H and L chain DNAs were cloned into expression vector pET-3a [Rosenberg, A. H. et al., (1987), Gene, 56, pp.125-135] forming pG1 (heavy chain, Figure III), and pK1 (light chain, Figure IV).

Induction of gene expression by IPTG resulted in high level expression of the expected proteins that reached approximately 140 mg/L of culture (5 g/L wet cell pellet weight) or about 35-40% of total cell protein. Pulse-labeling experiments indicated that these proteins represented 90-100% of total protein synthesized during the induction period. The identities of the induced proteins were confirmed by Western blotting using peptide-derived antisera and by NH$_2$-terminal sequence analysis. Each of the expressed antibody fragments accumulated as insoluble inclusion bodies following IPTG induction and were solubilized in the presence of 6M guanidine-HCl and renatured under dilute conditions in buffer containing reduced and oxidized glutathione. Recombinant Fab were subsequently assembled by mixing equal amounts of the H and L chains from broken cell pellets and subjecting them to reduction, reoxidation, and dialysis. Final purification was carried out by HPLC gel filtration, and purity (>95%) was visually determined by SDS-PAGE.

To determine whether the recombinant Fab's were biologically active, their ability to block binding of $^{35}$S-methionine-labeled HRV-15 to HeLa cell membranes was measured (Figure VI). The recombinant Fab, and Fab fragments proteolytically derived from intact antibody, were biologically active after renaturation, showing half-maximal inhibitions of virus binding at 0.3 and 0.2 pmoles/40 uL, respectively. Intact antibody displayed half-maximal inhibition at 0.1 pmoles/40 uL reaction.

To reduce the complexity of the expressed proteins, recombinant single-chain Fv in which the CO$_2$H-terminus of the L variable domain (V$_L$) was convalently linked to the NH$_2$-terminus of the H variable domain (V$_H$), were constructed. It is readily apparent to those skilled in the art, that for single chain Fv the order of the H and L variable domains may be exchanged, in that the CO$_2$H-terminus of the H variable domain may be covalently linked to the NH$_2$-terminus of the L variable domain.

In the first such recombinant, pSCV1 (Figure V), L chain Glu-124 was directly linked to H chain Glu-1. In an effort to lengthen the peptide linker while maintaining sufficient flexibility for proper V$_L$-V$_H$ interaction, two additional recombinants, pSCV2 and pSCV3, were constructed. These encode the flexible linker sequence Gly-Gly-Gly-Gly-Ser as a monomer (pSCV2), or dimer (pSCV3) respectively, between L chain Ser-122 and H chain Glu-1. These recombinants also expressed proteins of the expected size (about 25 KD) as visualized by SDS-PAGE, although in slightly lower amounts (about 50 mg/L culture) than the L and H Fab chains. The single chain Fv were solubilized, renatured, and purified as described above for Fab. Final recoveries of recombinant Fab and single chain Fv were 4-5 mg/liter and 10-12 mg/liter of culture, respectively.

The protein encoded by pSCV1 was the least active of the single chain Fv, achieving half-maximal blocking at 4 pmoles/40 uL reaction. The proteins encoded by pSCV2 and pSCV3 had approximately 3-fold higher activity, showing half-maximal inhibition at 1.5 pmoles. There was no significant difference in activity between single chain Fv with monomeric or dimeric Gly$_4$Ser linkages, suggesting that there is considerable flexibility in the allowable lengths of the interdomain linker peptides. Therefore, it is readily apparent to those skilled in the art that minor variation in the length of the linking moiety will not result in the formation of single chain Fv with substantially different or novel properties or activities.

The individual H and L chains which comprise the Fv portion of the antibody molecule, may be expressed individually, and subsequently assembled into Fv in the same manner as was done for Fab, using the same procedures for solubilization, renaturation, and purification. It is therefore not essential that the single chain Fv of the present invention were expressed as one contiguous peptide molecule.

HeLa cell monolayers were pretreated individually with each of the antibody portions, diluted serially, and subsequently infected with HRV-14 as described in Example 5. After a 24 hour incubation at 34°C, cell monolayers were examined for evidence of cytopathic effects resulting from the cytolytic infection of HRV (see Figure VI). Intact 1A6 was protective at a concentration as low as 84 ng/mL (0.6 nM). Both recombinant Fab, and native Fab fragment proteolytically derived from intact antibody, were protective down to 740 ng/mL (16 nM). The single chain Fv showed significant activity, and were protective at concentrations above the 2.2-6.7 ug/mL (84-250 nM) range.

As originally expressed, the H and L chains each contained five cysteine residues. Four of the cysteine residues in each chain normally participate in intrachain disulfide bridges to form immunoglobulin domains, while the fifth is involved in interchain disulfide bonding to covalently link the H and L chains.

Oligonucleotide-directed mutations were introduced into the L chain cDNA clone to delete Cys-214, and in the H chain cDNA to change the Cys-235 to an Ala. These modifications delete the disulfide bond which normally links the H and L chains.

Following expression and renaturation, the disulfide-minus Fab (Fab(-SS)) exhibited the same retention time on HPLC gel filtration as the natural and recombinant Fab suggesting a noncovalent interaction between H and L chains. However, characterization by nonreducing SDS-PAGE demonstrated that the H and L chains of Fab(-SS) migrated as monomers, while the control proteolytically derived Fab and recombinant Fab migrated as dimers. This result indicates that the H and L chains of Fab(-SS) are held together by noncovalent interactions. Fab(-SS) were indistinguishable from the wild type recombinant Fab in the virus binding assay (Figure VI), demonstrating that the interchain disulfide bond is dispensable for antibody-antigen binding.

These results show that recombinantly produced subportions of immunoglobulin chains specific for ICAM-1 can be abundantly expressed in E. coli and efficiently purified and renatured into portions of the antibody molecule which maintain biological activity, in that the recombinant antibody portions bind to ICAM-1.

It is understood that the novel amino acid sequences of the present invention, encoding recombinant portions of MAb's specific for ICAM-1 are examples of a range of amino acid sequences encoding recombinant portions of antibody molecules which can bind to ICAM-1. Other variant amino acid sequences may have the same effect of binding to ICAM-1. For example, conservative amino acid substitutions in the sequences of the recombinant portions of antibody molecules of the present invention will not result in any substantial or novel modification of the principles and practice of the present invention. Conservative substitutions are known; see Elfassi, E. et al., J. Theor. Biol., 121, pp 371 (1986). Alternatively, minor insertions or deletions within, or at either terminus, of the recombinant proteins of the present invention will not affect the principles and practice of the present invention. It will be understood that the recombinant portions of antibody molecules which can bind to ICAM-1 in this application, includes any such variations in amino acid sequence, whether by conservative amino acid substitution, insertion deletion, or other process, provided that the recombinant portion of an antibody molecule, after purification, is immunochemically reactive with ICAM-1.

The following examples illustrate the present invention without, however, limiting the same thereto.

## EXAMPLE 1

### Virus Growth and Purification

HRV-14 was obtained from the American Type Culture Collection (Rockville, MD), ATCC VR-284 and plaque purified by standard techniques [Yin et al., (1973), J. Virol., 12, pp.108-113]. Plaque purified HRV was propagated on HeLa R19 cells, a subclone of HeLa cells derived by standard procedures [Coller et al., (1983), Hybridoma, 2, pp. 91-96]. Cells were routinely propagated in growth medium (GM): McCoy's 5A medium containing 10% fetal calf serum, 30 mM MgCl$_2$ and 20 units/mL penicillin and 20 $\mu$g/mL streptomycin. HeLa R19 monolayers were infected at a MOI of 1 and harvested when cells detached freely from the flask surface (usually 16-24 hours). The cell suspension was frozen and quickly thawed to release virus particles from the cells. After clarification by centrifugation at slow speed (4000 x g for 5 minutes at 4°C), polyethylene glycol-6000 (PEG-6000) and NaCl were added to concentrations of 7% and 2.2%, respectively, and the mixture stirred at 4°C for 4-16 hours. The precipitated virus was recovered by centrifugation (10,500 x g for 15 minutes at 4°C) and resuspended in 10 mM R-buffer (10 mM TRIS-HCl, pH 7.5, 1 mM EDTA [ethylenediamine tetraacetic acid], 0.2 M NaCl, 50 mM MgCl$_2$ and 10% glycerol). Sodium deoxycholate and polyoxyethylene (9) octaphenol were added to concentrations of 0.3% and 0.6%, respectively, incubated for 30 minutes on ice, and the suspension was clarified by centrifugation (4000 x g for 5 minutes at 4°C). The supernatant (7.5 mL) was layered over a 5 mL linear density gradient of 40-60% (w/v) metrizamide in R-buffer lacking glycerol. Isopycnic banding of the virus sample was achieved by centrifugation for 24 hours at 150,000 x g. Virus bands were generally the densest of the visible bands in the gradient and were harvested manually by bottom puncture. After dilution 3-fold in R-buffer, the virus was repelleted by centrifugation at 200,000 x g for 2 hours.

EXAMPLE 2

Immunization of Mice With HeLa R19 Cells

Adult BALB/C mice were injected intraperitoneally (IP) with a solution containing HeLa R19 cells which were recovered from tissue culture monolayers by treatment with phosphate buffered saline (PBS) containing 50 mM EDTA for 20 minutes at 37°C. Each mouse received $3 \times 10^6$ cells suspended in a volume of 0.5 mL of complete Freund's adjuvant. After 38 days, mice were reinoculated IP with $1 \times 10^7$ HeLa R19 cells in a volume of 0.5 mL of incomplete Freunds's adjuvant.

Three mice whose sera was positive for antibodies which protected cells against HRV-14 infection (See Example 14) were primed on day 116, 4 days prior to the cell fusion, by injection of two $OD_{280}$ units/mouse. Sera collected at death of the animals showed the presence of antibody capable of protecting HeLa cells from HRV-14 infection.

In another experiment three adult BALB/C mice were immunized using the following schedule: 2 IP inoculations using cell membrane preparations on day 1 and day 42 followed by a final intradermal inoculation of membranes plus incomplete Freund's adjuvant on day 214. Three days later the cell fusion was carried out as described in Example 4.

EXAMPLE 3

Preparation of HeLa Cell Membranes

HeLa R19 cell monolayers were treated with PBS containing 50 mM EDTA for 20 minutes at 37°C. Cells were collected by low speed centrifugation and washed with PBS three times. Cells were pelleted and resuspended in 10 mM sodium phosphate buffer, pH 7.0 ($8 \times 10^7$ cells/mL) and placed on ice for 25 minutes. Cells were then disrupted by 20 strokes in a Dounce homogenizer, and nuclei were removed by centrifugation (1,000 x g for 5 minutes at 4°C). The supernatant was discarded and the membranes were pelleted at 142,000 x g for 1 hour at 4°C. The supernatant was discarded and the crude membrane pellet was dissolved in PBS at 42 $OD_{280}$ units/mL and stored at -70°C.

EXAMPLE 4

Production of Hybridomas by Fusion of Immune Mouse Spleen Cells with Mouse Myeloma Cells

Mouse myeloma cells (SP 2/0) were grown from frozen seed stock in HT media. Each 100 mL of HT medium contained 66 mL of Dulbecco's Modified Eagle's Medium; 20 mL fetal calf serum (FCS); 10 mL of NCTC 109 with Eagle's balanced salts (Gibco); 2 mL of 200 mM L-glutamine; 1 mL of a solution containing 408 mg hypoxanthine and 114 mg thymidine in 300 mL of distilled $H_2O$; and 1 mL of OPI stock (1.5 g cisoxalacetic acid 0.5 g pyruvic acid, 2000 units bovine insulin in 100 mL $H_2O$); and 0.2 mL of a penicillin (10,000 μg/ml) and streptomycin (10,000 μg/mL) mixture. The spleens from mice immunized with HRV-14 (Example 1) were removed after cervical dislocation and placed in serumless HT medium at room temperature. The spleens were placed in a plastic petri dish and the cells were teased from the spleen with a plastic scraper. The plates were washed with 5 mL of serumless HT medium and the cells were pooled into a 15 mL plastic conical tube; large particles were allowed to settle for one minute. The supernatant was then transferred to a 15 mL plastic round bottom tube and the cells were pelleted by a 10 minute centrifugation at 350 x g at room temperature. The supernatant was discarded, the cells were resuspended in serumless HT medium (10 mL/2 spleens) and the total number of viable cells was determined by trypan blue exclusion.

The number of viable SP 2/0 myeloma cells was also determined, and the cells were combined using ten-fold more spleen cells than myeloma cells (i.e. into a 50 cc screw cap plastic tube were placed $2 \times 10^8$ spleen cells and $2 \times 10^7$ SP 2/0 cells). The cells were pelleted by centrifugation for 10 minutes at 350 x g, and the cell pellet was resuspended in 10 mL of serumless HT medium. This procedure was repeated twice, and after the final resuspension in 10 mL of serumless HT medium the cells were transferred to a 15 mL round bottom tube. The cells were pelleted at 350 x g for 10 minutes.

Polyethylene glycol (PEG/DMSO/HT molecular weight average = 1000d) was liquified at 45°C and combined with serumless HT medium to a concentration of 35% PEG (vol/vol). The PEG/HT medium mixture was sterilized by passage through a 0.22 micron membrane filter fitted on a 3 mL syringe; the PEG was then maintained at 37°C. Dimethylsulfoxide (DMSO) was added to the PEG/medium mixture to a final concentration of 5%. The PEG/DMSO/HT medium mixture was added dropwise to the spleen-myeloma cell pellet, using 0.8 mL for $2 \times 10^8$ cells, while gently resuspending the cells by tapping the side of the culture tube and swirling. The cells were centrifuged at 250 x g at room temperature so that the total contact time of the cells with PEG was 6 minutes. The supernatant was aspirated and the cells were resuspended in 10 mL of HT medium. The cells were pelleted (350 x g for 10 minutes) and gently resuspended in HT medium to a final concentration of $3.5 \times 10^5$ cells/mL. The cells were then plated in 96 well microtiter plates using 0.1 mL/well, and incubated at 37°C in a water-jacketed incubator with 5% $CO_2$ and 96% humidity.

After 24 hours 0.1 mL of HAT medium (HT medium plus aminopterin at 0.352 mg/liter) was added to each well. The wells were refed with 0.1 mL of fresh HAT medium every 4 to 5 days.

Some cells from culture wells which were at a confluency of 15% or greater (usually 40 to 80%) were tested for production of antibodies to HRV-14. In other experiments some cells from growth-positive wells which were at a confluency of 15% or greater were tested for production of antibodies which block attachment of HRV-14 to cellular receptors in a cell protection assay described in Example 5.

Those cells having high activity by the above assays were subcloned by two cycles of limiting dilutions. The purified mAb from these subclones were characterized as to immunoglobulin subtype and molecular weight by standard procedures.

EXAMPLE 5

Cell Protection Assays

Protection assays utilized 48-well cluster plates of HeLa R-19 cells ($1.25 \times 10^5$ cells/well). After removal of media, monolayers were incubated with 0.1 mL of fresh medium, or hybridoma tissue culture fluid for 1 hour at 34°C. After incubation, $2 \times 10^5$ PFUs of HRV-14 in 50 $\mu$L of media were added to the treated monolayer and the cells incubated 16-24 hours at 34°C in a $CO_2$ incubator. Wells were scored as positive if no evidence of cytopathic effect was evident compared to controls, and three protective antibodies were identified. Further assays were done to determine if these mAb would protect cells against infection with other serotypes of HRV. Positive protection results were obtained with 90 different serotypes of HRV, a clinical isolate of unknown serotype, and coxsackie virus types A13, A18 and A21 (See Table I). All of these serotypes are known to share the same cellular receptor. Eleven serotypes of HRV which share a different receptor were not blocked by the mAb. These mAb's also failed to protect cells against Sabin Type 1 polio virus and coxsackie viruses Types B2 and B3.

EXAMPLE 6

Purification of Monoclonal Antibodies From Tissue Culture Media and Ascites Fluid

The mAb's were purified from tissue culture fluid taken from cultures of hybridoma cells producing an individual antibody. The following procedure was employed and is essentially that described by Emini, et al., J.Virol. 43:997-1005, 1982. One liter of tissue culture fluid from each particular hybridoma cell line was sequentially filtered through a Whatmann 1MM filter paper, a glass wool containing column, and finally a Sepharose 6B column. The pH was adjusted to about 8.0 by the addition of 100 mM TRIS-HCl, pH 8.0, and then passed through a Protein-A Sepharose column (4 to 6 mL bed volume per 2 liters of fluid). The column was washed with 10 bed volumes of 10 mM TRIS-HCl, pH 8.0. Glycine (100 mM), pH 3.0, was then added to elute the bound immunoglobulins from the column. Fractions were collected into tubes containing 100 mM TRIS-HCl, pH 8.0, to stabilize the antibody. Peak protein fractions from the column were pooled, dialyzed against distilled $H_2O$ and used as whole antibody or were treated further to obtain Fab.

To obtain Fab of the individual mAb, 10 to 15 mg of mAb purified using protein A columns, as described above, was lyophilized and then dissolved in 100 mM sodium phosphate, 10 mM cysteine, pH 7.2 (2 mL/20 mg antibody). Papain, 10-15 units per mg, was added to a ratio of 1:100, enzyme to antibody by weight. This reaction was incubated overnight for 16 hours at 37°C and terminated by the addition of iodoacetamide to a final concentration of 30 mM. The digested mAb was then chromatographed over cross-linked dextran (Sephadex G-75) column (2.5 x 20 cm) at 4°C and the fractions were monitored for absorbance at 280 nm. The second $A_{280}$ peak, which contains the majority of Fab, was pooled and then passed through a protein A-sepharose column to eliminate any contaminating Fc fragments or undigested mAb. Peak fractions of Fab from the protein A column were pooled, dialyzed against distilled $H_2O$, lyophilized in 200 $\mu$g aliquots, and stored at -80° C.

EXAMPLE 7

Determination of NH$_2$-terminal Amino Acid Sequence of Antibodies

The NH$_2$-termini of the IA6, 2C2 and 18B9 H and L chains were sequenced to provide amino acid data for the synthesis of oligonucleotide probes to be used in cDNA cloning of the respective genes. Antibody H and L chains were separated by electrophoresis through an 8% SDS polyacrylamide gel under reducing conditions. The protein was transferred onto polybrene-coated glass fiber filters according to the method of Vandekerckhove et al., (1985), Eur.J.Biochem., 152, pp. 9-19. Strips containing each chain were cut out and the NH$_2$-terminal sequence was determined by the method of Hewick et al., 1981 using a gas-phase microseqenator (Applied Biosystems).

The amino acid sequence of the H and L chains of mAb 1A6, 2C2, and 18B9 are shown in Tables 3, 4, and 5, respectively.

## TABLE 3

### Amino Acid Sequence Of mAb 1A6 H Chain Variable Region:

NH$_2$-Glu Val Gln Leu Gln Gln Ser Gly Ala Glu Leu Val Lys Pro Gly Ala Ser

Val Lys Leu Ser Cys Thr Ala Ser Gly Phe Asn Ile Lys Asp Thr Tyr Ile

His Trp Met Lys Gln Arg Pro Glu Gln Gly Leu Glu Trp Ile Gly Arg Ile

Asp Pro Ala Asn Asp Asn Thr Ile Tyr Asp Pro Lys Val Gln Gly Lys Ala

Thr Met Thr Ala Asp Thr Ser Ser Ans Thr Ala Tyr Leu Gln Leu Ser Ser

Leu Thr Ser Glu Asp Thr Ala Val Tyr Tyr Cys Thr Thr Ser Gly Tyr Trp

Phe Ala Tyr Trp Gly Gln Gly Thr Leu Val Thr Val Ser Ala-CO$_2$H

### Amino Acid Sequence Of mAb 1A6 L Chain Variable Region:

NH$_2$-Asp Ile Val Leu Thr Gln Ser Pro Ala Thr Leu Ser Val Thr Pro Gly Asp

Ser Val Ser Leu Ser Cys Arg Ala Ser Gln Ser Ile Ser Asn Asn Leu His

Trp Tyr Gln Gln Lys Ser His Glu Ser Pro Arg Leu Leu Ile Lys His Ala

Ser Gln Ser Ile Ser Gly Ile Pro Ser Arg Phe Ser Gly Ser Gly Ser Gly

Thr Asp Phe Thr Leu Ser Ile Asn Ser Val Glu Thr Glu Asp Phe Gly Met

Phe Phe Cys Gln Gln Ser Asn Ser Trp Pro Tyr Thr Phe Gly Gly Gly Thr

Lys Leu Glu Ile Lys Arg-CO$_2$H

## TABLE 4

Amino Acid Sequence Of mAb 2C2 H Chain Variable Region:

NH$_2$-Glu Val Gln Leu Gln Gln Ser Gly Ala Glu Leu Val Lys Pro Gly Ala

Ser Val Lys Leu Ser Cys Thr Ala Ser Gly Phe Asn Ile Lys Asp Thr Tyr

Met His Trp Val Lys Gln Arg Pro Glu Gln Gly Leu Glu Trp Ile Gly Arg

Ile Asp Pro Ala Asn Ile Asn Thr Lys Tyr Asp Pro Lys Phe Gln Gly Lys

Ala Thr Leu Thr Ala Asp Thr Ser Ser Asn Thr Ala Tyr Leu Gln Leu Ser

Ser Leu Thr Ser Glu Asp Thr Ala Val Tyr Tyr Cys Ser Arg Ser Asp Phe

Gly Ser Ser Tyr Val Asp Ala Tyr Trp Gly Arg Gly Thr Leu Val Thr Val

Ser Ala Ala Lys Thr Thr Pro Pro-CO$_2$H

Amino Acid Sequence Of mAb 2C2 L Chain Variable Region:

NH$_2$-Asp Ile Gln Leu Thr Gln Ser Pro Ala Ser Leu Ala Val Ser Leu Gly

Gln Arg Ala Thr Ile Ser Cys Arg Ala Ser Gln Ser Val Ser Thr Ser Thr

Phe Ser Tyr Met His Trp Tyr Gln Gln Lys Pro Gly Gln Pro Pro Lys Leu

Leu Ile Lys Phe Ala Ser Asn Leu Glu Ser Gly Val Pro Ala Arg Phe Ser

Gly Ser Gly Ser Gly Thr Asp Phe Thr Leu Asn Ile His Pro Val Glu Glu

Asp Asp Thr Ala Thr Tyr Tyr Cys Gln His Ser Trp Glu Ile Pro Phe Thr

Phe Gly Ser Gly Thr Lys Leu Glu Ile Lys Arg Ala Asp Ala Ala Pro Thr

Val Ser Ile Phe Pro Pro Ser-CO$_2$H

12

EP 0 459 577 A2

## TABLE 5

Amino Acid Sequence Of mAb 18B9 H Chain Variable Region:

$NH_2$-Glu Val Gln Leu Gln Gln Ser Gly Pro Glu Leu Val Lys Pro Gly Ala
Ser Val Lys Met Ser Cys Lys Ala Ser Gly His Thr Phe Thr Ser Phe Val
Ile His Trp Leu Lys Gln Lys Pro Gly Gln Gly Leu Glu Trp Ile Gly Tyr
Val Asn Pro Tyr Val Asp Asp Ser Lys Tyr Asn Glu Lys Phe Lys Gly Lys
Ala Thr Leu Thr Ser Asp Lys Ser Ser Ser Thr Ala Tyr Met Glu Leu Ser
Ser Leu Thr Ser Glu Asp Ser Ala Val Tyr Tyr Cys Ala Ser Gly Asn Tyr
Tyr Ala Phe Phe Asp Tyr Trp Gly Gln Gly Thr Thr Leu Thr Val Ser Ser
Ala Lys Thr Thr Pro Pro-$CO_2$H

Amino Acid Sequence Of mAb 18B9 L Chain Variable Region:

$NH_2$-Asp Ile Gln Leu Thr Gln Ser Pro Ala Ser Leu Ala Val Ser Leu Gly
Gln Arg Ala Thr Ile Ser Tyr Arg Ala Ser Lys Ser Val Ser Thr Ser Gly
Tyr Ser Tyr Met His Trp Asn Gln Gln Lys Pro Gly Gln Pro Pro Arg Leu
Leu Ile Tyr Leu Val Ser Asn Leu Glu Ser Gly Val Pro Ala Arg Phe Ser
Gly Ser Gly Ser Gly Thr Asp Phe Thr Leu Asn Ile His Pro Val Glu Glu
Glu Asp Ala Ala Thr Tyr Tyr Cys Gln His Ile Arg Glu Pro Tyr Thr Phe
Gly Gly Gly Thr Lys Leu Glu Ile Lys Arg Ala Asp Ala Ala Pro Thr Val
Ser Ile Phe Pro Pro Ser-$CO_2$H

13

## TABLE 6

### 10. Amino Acid Sequence Of Single Chain Fv From pSCV1:

Asp Ile Val Leu Thr Gln Ser Pro Ala Thr Leu Ser Val Thr Pro Gly Asp

Ser Val Ser Leu Ser Cys Arg Ala Ser Gln Ser Ile Ser Asn Asn Leu His

Trp Tyr Gln Gln Lys Ser His Glu Ser Pro Arg Leu Leu Ile Lys His Ala

Ser Gln Ser Ile Ser Gly Ile Pro Ser Arg Phe Ser Gly Ser Gly Ser Gly

Thr Asp Phe Thr Leu Ser Ile Asn Ser Val Glu Thr Glu Asp Phe Gly Met

Phe Phe Cys Gln Gln Ser Asn Ser Trp Pro Tyr Thr Phe Gly Gly Gly Thr

Lys Leu Glu Ile Lys Arg Ala Asp Ala Ala Pro Thr Val Ser Ile Phe Pro

Pro Ser Ser Glu Glu Glu Val Gln Leu Gln Gln Ser Gly Ala Glu Leu Val

Lys Pro Gly Ala Ser Val Lys Leu Ser Cys Thr Ala Ser Gly Phe Asn Ile

Lys Asp Thr Tyr Ile His Trp Met Lys Gln Arg Pro Glu Gln Gly Leu Glu

Trp Ile Gly Arg Ile Asp Pro Ala Asn Asp Asn Thr Ile Tyr Asp Pro Lys

Val Gln Gly Lys Ala Thr Met Thr Ala Asp Thr Ser Ser Asn Thr Ala Tyr

Leu Gln Leu Ser Ser Leu Thr Ser Glu Asp Thr Ala Val Tyr Tyr Cys Thr

Thr Ser Gly Tyr Trp Phe Ala Tyr Trp Gly Gln Gly Thr Leu Val Thr Val

Ser Ala.

## EXAMPLE 8

### Isolation and purification of hybridoma mRNA

Hybridoma cells (IA6, 2C2, and 18B9) growing in suspension culture were pelleted from the growth medium by centrifugation at 1,000 x g for 10 minutes at 4°C. The pelleted cells ($10^9$) were swollen in 20 mL hypotonic buffer (10 mM NaCl, 5 mM MgCl, 50 mM TRIS-HCl, pH 7.5, 5% sucrose, 0.5 mg/mL Heparin) for 30 minutes on ice, then 10 mL of 110 mM NaCl, 7.5 mM $MgCl_2$, 7.5% sucrose, 75 mM Tris-HCl, pH 7.5, 0.5 mg/mL Heparin was added and the cells were broken in a dounce homogenizer. Cell debris and nuclei were pelleted at 1,000 x g for 2 minutes at 4°C. The supernatant was treated with 0.25% NaDoc and 0.50% NP-40, then mitochondria were pelleted at 4000 x g for 10 minutes. Polysomes were pelleted from the cleared supernatant by centrifugation through a 2.5 mL sucrose cushion (1 M Sucrose, 50 mM NaCl, 5 mM $MgCl_2$, 20 mM Tris-HCl, pH 7.5) at 200,000 x g for 60 minutes at 4°C. Polysomal pellets were resuspended in 10 mM TRIS-HCl, pH 7.5, 1 mM EDTA, 0.3% lithium dodecyl sulfate, and then treated with 1 mg proteinase-K at 37°C for 10 minutes.

RNA was heat denatured for 1 minute in a boiling water bath and quick-chilled on ice. NaCl was added to 0.5M and the mixture was chromatographed on a column containing 0.6g oligo(dT)-cellulose, (Type II, Collaborative Research) in 10mM TRIS-HCl, pH 7.5, 1mM EDTA, 0.3% lithium dodecyl sulfate, 0.5M NaCl. Poly-(A)$^+$ mRNA was eluted from the column in hot distilled water (56°C).

## EXAMPLE 9

### Preparation of cDNA libraries

Double-stranded cDNA was synthesized from poly(A)$^+$ RNA by the method of Gubler, U. et al., (1983) Gene, 25, pp. 263-69, using Amersham's cDNA Synthesis Plus reagents. The synthesis was performed by first priming the RNA with oligo-dT to obtain constant region cDNA, then with internal oligonucleotides 12 and 13 (Table II) complementary to the known constant region, or a commercial variable region primer (C-gamma 15, Pharmacia and oligonucleotide 14, Table II) to obtain the full-length cDNA. The double-stranded cDNA was blunt-end ligated to EcoRI linkers (New England Biolabs) and then digested with EcoRI. Excess linkers were removed by chromatography on a Sephadex G-150 (Pharmacia) column. A phage library was made by ligating the purified cDNA into lambda ZAP dephosphorylated EcoRI arms (Stratagene) and packaged in Stratagene Gigapack Plus extracts according to directions supplied by the manufacturer. The appropiate bacterial strain was infected at 37°C for 10 minutes with the phage library and then plated at 35,000 PFU/150 mm Luria agar plate, and incubated at 37°C overnight.

EXAMPLE 10

Cloning of Anti-ICAM-1 antibody cDNA clones from cDNA libraries

The cDNA libraries were screened by filter hybridization according to the method of Benton, W.D., and Davis, R.W., (1977), Science, 196, pp. 180-182. Multiple lifts of phage DNA onto nitrocellulose filters (Scheicher and Scheull) were hybridized to $^{32}$P-labelled oligonucleotide probes in 6X SSC, 5X Denhardt's, 0.5% SDS, 50mM NaPo$_4$, and 0.2mg/ml salmon sperm DNA, overnight at 10°C below the calculated Tm of the oligonucleotide used.

The sequences of the oligonucleotide probes were predicted from both the conserved DNA sequences in the constant regions of gammaglobulin-1 H chains (oligonucleotide 15, Table II) and lambda L chains (oligonucleotide 16, Table II), and using amino acid sequences obtained from the L and H chains of the mAb. Following hybridization, the filters were washed in 6X SSC, 0.5% SDS for 5 minutes at 23°C, then for 10 minutes at the hybridization temperature. Hybridization positive phage were plaque purified by dilution plating and rescreening. Plasmids were rescued from the phage and the EcoRI inserts were cloned into pGem vectors (Promega Biotech) to yield plasmids p1A6L (light chain) and p1A6H (heavy chain).

EXAMPLE 11

## Expression of Anti-ICAM-1 Antibody Light Chains in E. coli

Plasmid p1A6L was linearized with ScaI, phenol-extracted, ethanol-precipitated, and amplified by polymerase chain reaction (PCR) using the Perkin-Elmer/Cetus GeneAmp DNA Amplification Reagent Kit, and following the manufacturer's recommended procedures. Priming was accomplished with synthetic oligonucleotides 1 and 2 (Table 2, Example 13). 25 cycles of amplification were performed in a Perkin-Elmer DNA Thermal Cycler, each cycle consisting of incubations at 94°C, 1 minute; 37°C, 2 minutes; and 72°C, 3 minutes. In each cycle, the 72° incubation was extended 2 seconds over the previous cycle.

The PCR-amplified DNA fragment was separated on a 0.7% SeaPlaque Agarose (FMC) gel, excised, melted at 65°, and purified on a NACS Prepac Column (BRL) following the procedures supplied by the manufacturer. The purified fragment was digested with NdeI and Bg1II, phenol-extracted, ethanol-precipitated, and ligated to an equimolar amount of plasmid pET-3a (10ug/mL final concentration) that had been digested with NdeI and BamHI and gel-purified as described above. After ligation using T4 DNA ligase for 15 hours at 4°C, 100 μL of competent E. coli AG1 (Stratagene) were transformed with 2 μL of the ligation mix according to the manufacturer's procedure, and plated on LB plates (containing, per liter: 10g Bacto-Tryptone, 5g Difco Yeast Extract, 5g NaCl, and 15g Bacto-Agar) supplemented with 100 ug/mL ampicillin.

After overnight incubation at 37°C colonies were transferred to nitrocellulose filter discs and prepared for colony hybridization. Colonies were hybridized against $^{32}$P-labelled oligonucleotide 3 (Table 2, Example 13) at 55°C, as previously described in Example 10. Filters were washed four times for 3 minutes each, with 6X SSC (0.9M NaCl, 0.09M Na Citrate) at 55°C, air dried, and autoradiographed. By DNA sequencing and restriction endonuclease mapping of plasmid DNA, the desired plasmid structure was confirmed.

The plasmid, designated pK1 (Figure III), was isolated and used to transform E. coli BL21 (DE3), made competent by the procedure of Hanahan. After overnight growth at 37°C on LB plates with 100 ug/mL ampicillin, a single colony was used to inoculate 500 mL of M9TB medium, containing per liter, 10g Bacto-Tryptone, 5.5g NaCl, 4g glucose, 6g Na$_2$HPO$_4$, 3g KH$_2$PO$_4$, 1g NH$_4$Cl, 120 mg MgSO$_4$, and 15 mg CaCl$_2$.2R$_2$O. Growth and IPTG-induction of the recombinant protein were as described previously, and expression was confirmed by SDS-PAGE.

EXAMPLE 12

## Expression of Anti-ICAM-1 Antibody Fd Fragments in E. coli

Plasmid p1A6H was linearized by ScaI digestion and the H chain coding region was PCR amplified as described in Example 11, except that synthetic oligonucleotides 11 and 4 (Table 2, Example 13) were used as primers. The product was gel-purified, digested with NdeI and Bg1II, and cloned into pET-3a as described in Example 11. Recombinant colonies were identified by colony hybridization against $^{32}$P-labelled oligonucleotide 5 (Table 2, Example 13) at 60°C as described in Example 10. The resulting pET-3a/Fd recombinant, pG1 (Figure IV), was transferred into E. coli strain BL21 (DE3) cells and expression was tested as described in Example 11.

EXAMPLE 13

## Expression of Single Chain Fv in E. coli

Plasmid pK1 was linearized with ScaI and PCR-amplified using oligonucleotides 1 and 6 (Table 2, Example 13) as primers. The product (Fragment 1) was digested with NdeI and XhoI and purified on a 1% SeaPlaque preparative agarose gel.

Plasmid pG1 was linearized with ScaI and PCR-amplified using oligonucleotides 7 and 10, 8 and 10, or 9 and 10 (Table 2, Example 13) as primers, and the products (fragments 2A, 2B, or 2C, respectively) were digested with XhoI and Bg1II, then purified on 1% SeaPlaque agarose gels.

The different single-chain Fv were generated by ligating equimolar mixtures of purified fragments 1 and 2A, 1 and 2B, or 1 and 2C, with an equimolar amount of plasmid pET-3a, previously digested with NdeI and BamHI. Recombinant plasmids were screened, transferred to E. coli BL21 (DE3) cells, and expression of the recombinant protein was determined as described in Example 11.

## TABLE 2

### Synthetic Oligodeoxynucleotide Sequences

1.  d(CGCATATGGATATTGTGCTAACTCAG)
2.  d(GCAGATCTTGGTGGTGGCGTCCTCAGGACCTTT)
3.  d(GATATTGTGCTAACTCAGTCTC)
4.  d(CCGGAGATCTGATCATTAACCACAATCCCTGGGCAC)
5.  d(AGCGGTGTGCACACCTTCCC)
6.  d(CTCACTGCAGGGTGGGAAGATGGATACAG)
7.  d(CCACCCTCGAGTGAGGAAGAAGTTCAGCTGCAGCAGTCCGGAGCAG)
8.  d(CCACCCTCGAGTGGTGGCGGTGGCTCTGAAGTTCAGCTGCAGCAGTCC
    GGAGCAG)
9.  d(CCACCCTCGAGTGGTGGCGGTGGCTCTGGCGGTGGCGGTTCCGAAGTT
    CAGCTGCAGCAGTCCGGAGCAG)
10.  d(CCGGAGATCTGATCATTATGCAGAGACAGTGACC)
11.  d(CGCATATGGAGGTTCAGCTGCAGCAGTCTGGG)
12.  d(CCTTAGGAGTCAGAGTAATGGTGAGC)
13.  d(ATCCCAGGGTCACCATGGAGTTAG)
14.  d(GGCCAGTGGATAGAC)
15.  d(GGGAAATAGCCCTTGACCAGGCATCC)
16.  d(GGTGGGAAGATGGATACAGTTGGTGC)
17.  d(GATATTGTGCTAACTCAGTCTGC)
18.  d(CCCAGGCTTCACAAGCTCAGCCCC)

EXAMPLE 14

Purification and Renaturation of Recombinant Anti-ICAM-1 Antibody Fragments

The cell pellet obtained from a 500 mL culture was resuspended in 15-20 mL of cell buffer at 4°C (0.05M Tris-HCl, 0.02M NaCl, 0.001M EDTA disodium salt, pH 8.1) containing 2μM TAME, 2μM pepstatin and 1mM PMSF. The cell suspension was passaged three times through a Stansted High Pressure Homogenizer. The pooled homogenate was centrifuged at 12,000 x g for 45 minutes at 4°C. The pellet was resuspended in cell buffer at approximately 100 mg of wet pellet weight per mL of buffer. Samples of the supernatant and pellet were analyzed by SDS-PAGE (12.5%). The antibody fragments were identified in the pellet fraction of the homogenate.

The pellet suspension (500 μl) was centrifuged for 5 minutes at 10,000 x g. The resulting pellet was resuspended in 300 μl of 5M guanidine-HCl, 0.1M TRIS-HCl, 0.001M EDTA, 0.02M NaCl, pH 8.2, and incubated at room temperature for 15 minutes with occasional vortexing, followed by centrifugation for 5 minutes at 10,000 x g. The protein concentration of the supernatant was determined by the Bradford procedure using IgG as the standard protein.

Proteins were suspended at 5 mg/mL in buffer I consisting of 6 M guanidine-HCl, 0.1 M TRIS, 0.02 M NaCl, 0.001 M EDTA, pH 8.3. $N_2$ was carefully flushed through the solution for 5 minutes, and then β-

17

mercaptoethanol (Sigma) was added to 0.1 M, and heated at 40° C for 45 minutes. A second aliquot of β-mercaptoethanol was then added to a final concentration of 0.2 M, and the sample was heated for an additional 45 minutes under N₂ atmosphere.

For recombinant Fab, the L and H chain pellets were resuspended in buffer I and equivalent amounts of both proteins were mixed. The protein samples were reduced by adding β-mercaptoethanol as described for single chain reduction.

Reoxidation and renaturation were achieved by diluting the reduced proteins to a concentration of 20μg/mL in buffer consisting of 1 M guanidine-HCl, 0.02 M TRIS-HCl, 0.02 M NaCl, 0.0001 M EDTA (disodium salt), pH 8.1. Reduced and oxidized glutathione were added at a final concentration of 2mM and 0.2mM, respectively, and the protein solution was incubated at 4° C for 16-20 hours in Spectra-por-2 dialysis membrane, with a molecular weight cutoff range of 12,000-14,000. The membranes were soaked at room temperature in distilled water for 30 minutes and washed thoroughly with deionized water before applying the protein solution.

Buffer exchange was carried out by dialysing against a 200-fold excess of PBS diluted 1:3 with deionized water. Two buffer changes were made every 4-5 hours followed by two buffer changes every 10-12 hours.

The dialyzed samples were concentrated to a volume of 1-2 mL. Concentrated samples were then centrifuged for 5 minutes at 10,000 x g and the supernatant was separated from the pellet. The supernatant containing renatured proteins was subjected to sizing column high performance liquid chromatography on a Biosil TSK-125 and a Biosil TSK-250 (Biorad) linked in series for optimal protein resolution. Columns were run using PBS sterilized by passage through a 0.22 micron filter. Peaks containing the antibody fragments were concentrated and analyzed by SDS-PAGE (12-5%) under reducing and non-reducing conditions.

EXAMPLE 15

Generation of ICAM-1 fragments.

The 5'-terminal noncoding region and the entire coding region of the ICAM-1 cDNA clone, pHRVr1 [Tomassini, J.E. et al., (1988) P.N.A.S. (USA) 86, pp.4907-11] was excised by digestion with restriction enzymes EcoRI and HindIII. The ICAM-1 coding region was then ligated into the transcription vector pGEM4Z, which was previously digested with EcoRI and HindIII, to form pGEM-ICAM. A full-length RNA copy of ICAM-1 was then generated by digestion of pGEM-ICAM with HindIII and subsequent transcription, in vitro, according to the procedure of the manufacturer (Promega Biotec). Similarly, successive carboxy-terminal truncations of ICAM-1 were generated by digesting pGEM-ICAM with BalI, NarI, MaeI, and RsaI, respectively, and then transcribing each in vitro.

Each RNA was extracted with phenol-chloroform and precipitated with ethanol in the presence of 2.75 M ammonium acetate.

A new construct encoding the domain 1 fragment of ICAM-1 was created by polymerase chain reaction amplification. DNA was primed at the 5' end by an oligonucleotide (5'-ACGCCAGGGTTTCCCAGTCACGA-3') homologous to the sense strand and located upstream of the SP6 promoter. DNA was also primed at the 3' end with an antisense oligonucleotide
(5'-CATCATCATCATGTACACGGTGAGGAAGGTTTTAGC-3')
homologous to ICAM-1 cDNA and containing 12 additional bases encoding four Met residues. The polymerase chain reaction was performed according to the directions supplied by the manufacturer with a DNA Thermal Cycler (Cetus-Perkin-Elmer).

EXAMPLE 16

Translation of ICAM-1 RNAs

Precipitated RNA was suspended in water at 1 mg/mL and stored at -20° C until it was used in translation reactions. Translation reaction mixtures in a 0.1 mL final volume, contained 0.02 mM of each essential amino acid except methionine, 0.05 mg of calf liver tRNA per mL, 800 U of RNasin per mL, 7.5 mCi of ³⁵S-methionine per mL (Amersham Corp.), 2 μg of each protease inhibitor (pepstatin A, antipain, and leupeptin) per mL, 16 U of canine pancreatic microsomal membranes per mL at an optical density of 280 nm (Dupont, NEN Research Products), 0.6 mL of rabbit reticulocyte lysate per mL of reaction mixture, 2 mM oxidized glutathione (added just prior to the addition of mRNA), and 5 μg of mRNA. Reactions were carried out at 30° C for 30 to 60 minutes, stopped by the addition of ribonuclease A to 0.1 mg/mL, and then

EP 0 459 577 A2

incubated an additional 10 minutes at 30°C. Radiolabeled protein was analyzed by autoradiography of translated products following SDS-PAGE.

EXAMPLE 17

Immunoprecipitation of translation products

Following translation, the reaction mixtures were cooled on ice, phenylmethylsulfonyl fluoride was added to 2 mM, and membrane vesicles were disrupted by adding Nonidet P-40 to 0.5%. 20 μL of a 50% slurry of protein A-agarose (Boehringer Mannheim Biochemicals) was added, and the mixture was rotated at 4°C for 10 minutes. The mixtures were pelleted at 10,000 x g for 2 minutes, and samples of the supernatants were transferred to tubes containing 0.5 μg of the mAb and were brought to a final volume of 0.1 mL with TRIS-buffered saline containing 0.25% Nonidet P-40. Following immunoprecipitation overnight at 4°C, 20 μL of protein A-agarose was added and this material was mixed for 30 min at 4°C. The agarose beads were gently pelleted and then washed twice with TRIS-buffered PBS containing 0.5% Nonidet P-40, and once with TRIS-buffered PBS alone. Pelleted agarose beads were suspended in 2x Laemmli protein gel loading buffer [Laemmli, U.K., (1970), Nature, 227, pp. 680-5] and were incubated at 100°C for 3 minutes. Eluted proteins were then analyzed by SDS-PAGE and autoradiography.

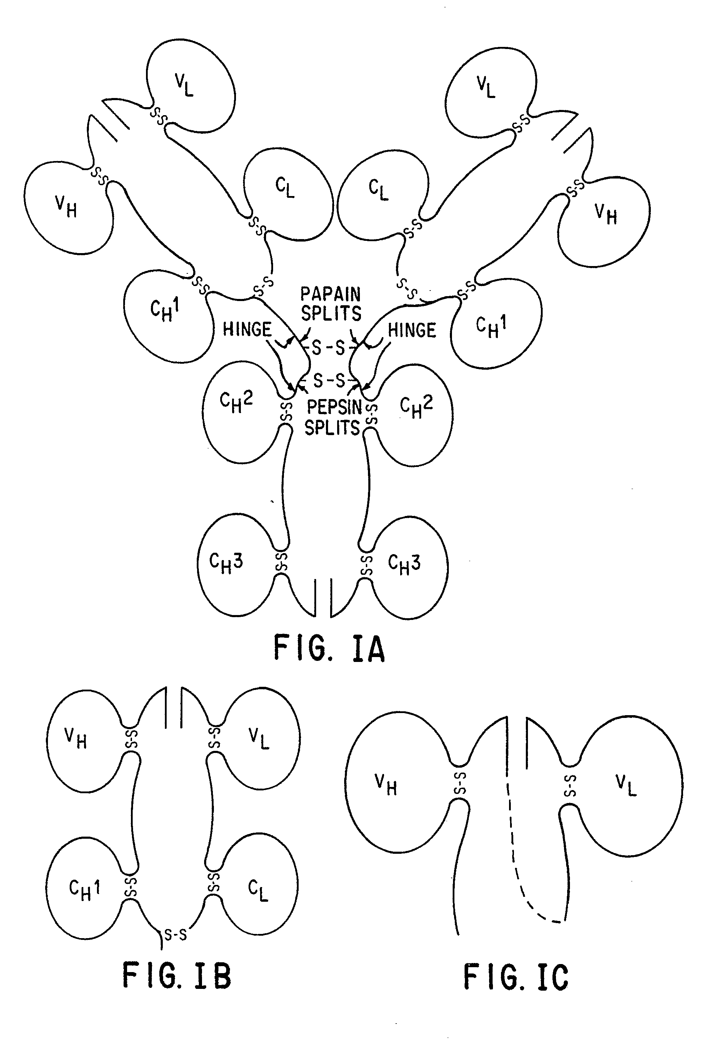

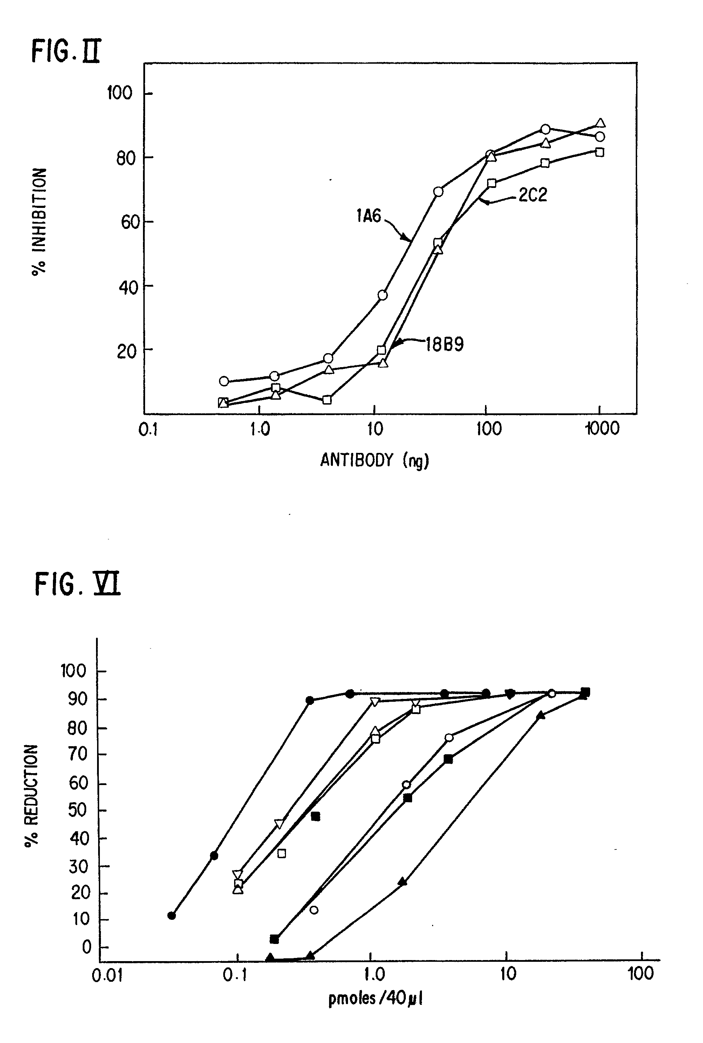

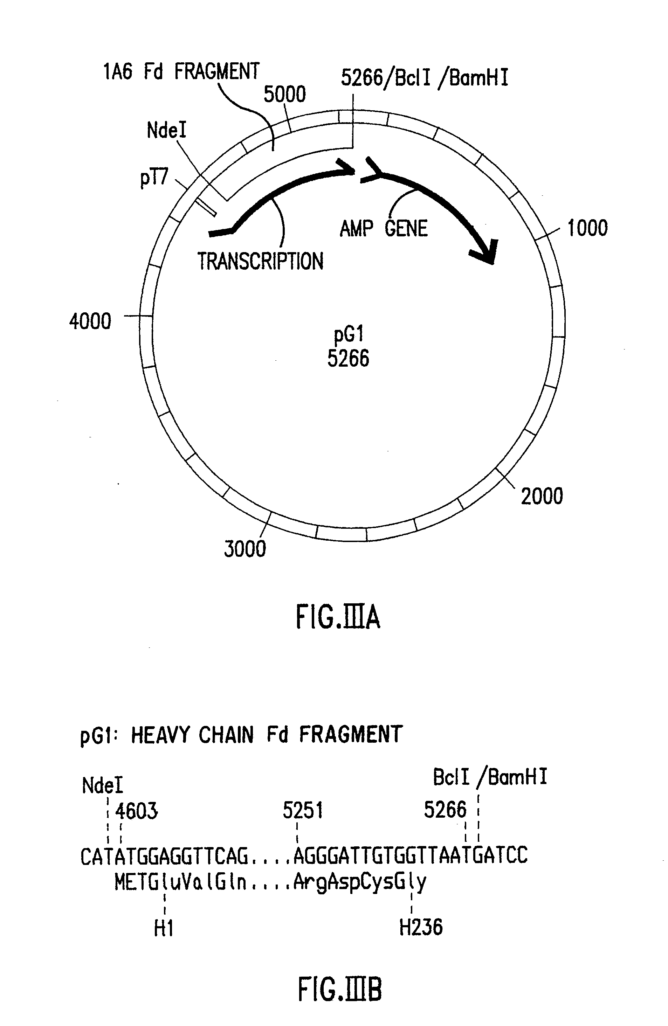

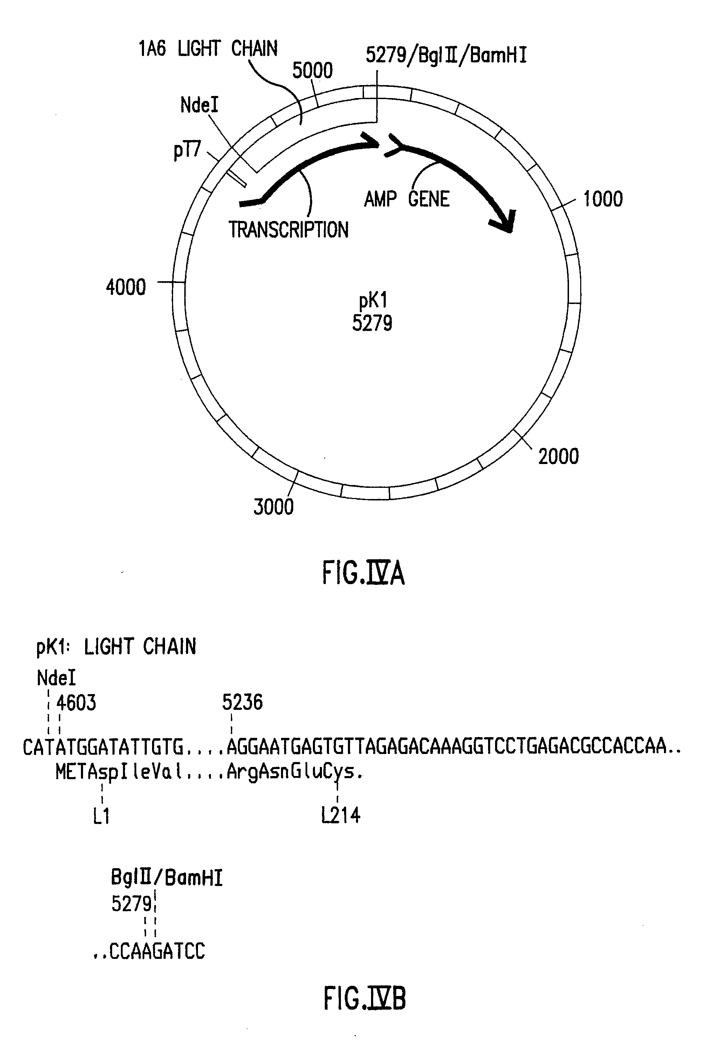

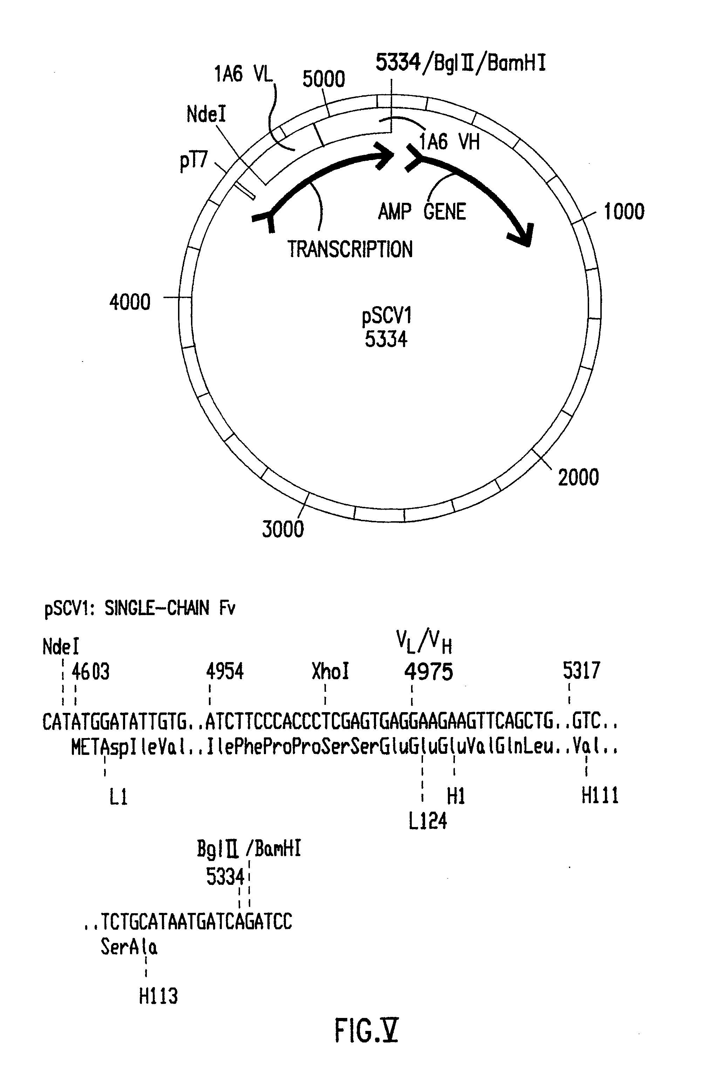

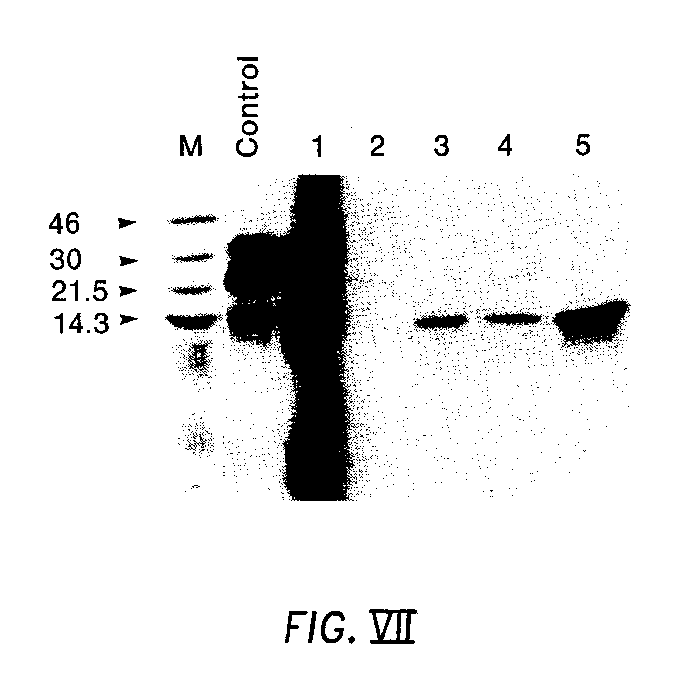

**Claims**

1. A peptide selected form the group consisting of:

(A)

Glu Val Gln Leu Gln Gln Ser Gly Ala Glu Leu Val Lys Pro Gly Ala Ser

Val Lys Leu Ser Cys Thr Ala Ser Gly Phe Asn Ile Lys Asp Thr Tyr Ile

His Trp Met Lys Gln Arg Pro Glu Gln Gly Leu Glu Trp Ile Gly Arg Ile

Asp Pro Ala Asn Asp Asn Thr Ile Tyr Asp Pro Lys Val Gln Gly Lys Ala

Thr Met Thr Ala Asp Thr Ser Ser Asn Thr Ala Tyr Leu Gln Leu Ser Ser

Leu Thr Ser Glu Asp Thr Ala Val Tyr Tyr Cys Thr Thr Ser Gly Tyr Trp

Phe Ala Tyr Trp Gly Gln Gly Thr Leu Val Thr Val Ser Ala;

(B)

Asp Ile Val Leu Thr Gln Ser Pro Ala Thr Leu Ser Val Thr Pro Gly Asp

Ser Val Ser Leu Ser Cys Arg Ala Ser Gln Ser Ile Ser Asn Asn Leu His

Trp Tyr Gln Gln Lys Ser His Glu Ser Pro Arg Leu Leu Ile Lys His Ala

Ser Gln Ser Ile Ser Gly Ile Pro Ser Arg Phe Ser Gly Ser Gly Ser Gly

Thr Asp Phe Thr Leu Ser Ile Asn Ser Val Glu Thr Glu Asp Phe Gly Met

Phe Phe Cys Gln Gln Ser Asn Ser Trp Pro Tyr Thr Phe Gly Gly Gly Thr

Lys Leu Glu Ile Lys Arg;

19

(C)

Glu Val Gln Leu Gln Gln Ser Gly Ala Glu Leu Val Lys Pro Gly Ala Ser Val

Lys Leu Ser Cys Thr Ala Ser Gly Phe Asn Ile Lys Asp Thr Tyr Met His Trp

Val Lys Gln Arg Pro Glu Gln Gly Leu Glu Trp Ile Gly Arg Ile Asp Pro Ala

Asn Ile Asn Thr Lys Tyr Asp Pro Lys Phe Gln Gly Lys Ala Thr Leu Thr Ala

Asp Thr Ser Ser Asn Thr Ala Tyr Leu Gln Leu Ser Ser Leu Thr Ser Glu Asp

Thr Ala Val Tyr Tyr Cys Ser Arg Ser Asp Phe Gly Ser Ser Tyr Val Asp Ala

Tyr Trp Gly Arg Gly Thr Leu Val Thr Val Ser Ala Ala Lys Thr Thr Pro Pro;

(D)

Asp Ile Gln Leu Thr Gln Ser Pro Ala Ser Leu Ala Val Ser Leu Gly Gln Arg

Ala Thr Ile Ser Cys Arg Ala Ser Gln Ser Val Ser Thr Ser Thr Phe Ser Tyr

Met His Trp Tyr Gln Gln Lys Pro Gly Gln Pro Pro Lys Leu Leu Ile Lys Phe

Ala Ser Asn Leu Glu Ser Gly Val Pro Ala Arg Phe Ser Gly Ser Gly Ser Gly

Thr Asp Phe Thr Leu Asn Ile His Pro Val Glu Glu Asp Asp Thr Ala Thr Tyr

Tyr Cys Gln His Ser Trp Glu Ile Pro Phe Thr Phe Gly Ser Gly Thr Lys Leu

Glu Ile Lys Arg Ala Asp Ala Ala Pro Thr Val Ser Ile Phe Pro Pro Ser;

(E)

Glu Val Gln Leu Gln Gln Ser Gly Pro Glu Leu Val Lys Pro Gly Ala Ser Val

Lys Met Ser Cys Lys Ala Ser Gly His Thr Phe Thr Ser Phe Val Ile His Trp

Leu Lys Gln Lys Pro Gly Gln Gly Leu Glu Trp Ile Gly Tyr Val Asn Pro Tyr

Val Asp Asp Ser Lys Tyr Asn Glu Lys Phe Lys Gly Lys Ala Thr Leu Thr Ser

Asp Lys Ser Ser Ser Thr Ala Tyr Met Glu Leu Ser Ser Leu Thr Ser Glu Asp

Ser Ala Val Tyr Tyr Cys Ala Ser Gly Asn Tyr Tyr Ala Phe Phe Asp Tyr Trp

Gly Gln Gly Thr Thr Leu Thr Val Ser Ser Ala Lys Thr Thr Pro Pro; and

(F)

Asp Ile Gln Leu Thr Gln Ser Pro Ala Ser Leu Ala Val Ser Leu Gly Gln Arg

Ala Thr Ile Ser Tyr Arg Ala Ser Lys Ser Val Ser Thr Ser Gly Tyr Ser Tyr

Met His Trp Asn Gln Gln Lys Pro Gly Gln Pro Pro Arg Leu Leu Ile Tyr Leu

Val Ser Asn Leu Glu Ser Gly Val Pro Ala Arg Phe Ser Gly Ser Gly Ser Gly

Thr Asp Phe Thr Leu Asn Ile His Pro Val Glu Glu Glu Asp Ala Ala Thr Tyr

Tyr Cys Gln His Ile Arg Glu Pro Tyr Thr Phe Gly Gly Gly Thr Lys Leu Glu

Ile Lys Arg Ala Asp Ala Ala Pro Thr Val Ser Ile Phe Pro Pro Ser.

20

EP 0 459 577 A2

2. A composition of peptides consisting essentially of peptide (A) and peptide (B) of Claim 1.

3. A composition of peptides consisting essentially of peptide (C) and peptide (D) of Claim 1.

4. A composition of peptides consisting essentially of peptide (E) and peptide (F) of Claim 1.

5. A peptide which has the amino acid sequence:

```
Asp Ile Val Leu Thr Gln Ser Pro Ala Thr Leu Ser Val Thr Pro Gly Asp
Ser Val Ser Leu Ser Cys Arg Ala Ser Gln Ser Ile Ser Asn Asn Leu His
Trp Tyr Gln Gln Lys Ser His Glu Ser Pro Arg Leu Leu Ile Lys His Ala
Ser Gln Ser Ile Ser Gly Ile Pro Ser Arg Phe Ser Gly Ser Gly Ser Gly
Thr Asp Phe Thr Leu Ser Ile Asn Ser Val Glu Thr Glu Asp Phe Gly Met
Phe Phe Cys Gln Gln Ser Asn Ser Trp Pro Tyr Thr Phe Gly Gly Gly Thr
Lys Leu Glu Ile Lys Arg Ala Asp Ala Ala Pro Thr Val Ser Ile Phe Pro
Pro Ser Ser Glu Glu Glu Val Gln Leu Gln Gln Ser Gly Ala Glu Leu Val
Lys Pro Gly Ala Ser Val Lys Leu Ser Cys Thr Ala Ser Gly Phe Asn Ile
Lys Asp Thr Tyr Ile His Trp Met Lys Gln Arg Pro Glu Gln Gly Leu Glu
Trp Ile Gly Arg Ile Asp Pro Ala Asn Asp Asn Thr Ile Tyr Asp Pro Lys
Val Gln Gly Lys Ala Thr Met Thr Ala Asp Thr Ser Ser Ans Thr Ala Tyr
Leu Gln Leu Ser Ser Leu Thr Ser Glu Asp Thr Ala Val Tyr Tyr Cys Thr
Thr Ser Gly Tyr Trp Phe Ala Tyr Trp Gly Gln Gly Thr Leu Val Thr Val
Ser Ala.
```

6. A peptide according to Claim 5 which corresponds to the Fv portion of an immunoglobulin molecule.

7. A peptide or composition of peptides which corresponds to the Fab or Fv portion of an immunoglobulin molecule which is specific for the cellular receptor of a respiratory virus.

8. The peptide or composition of peptides according to Claim 7, which is specific for the cellular receptor of a rhinovirus.

9. A peptide or composition of peptides according to Claim 8, which is capable of blocking the attachment of serotypes of the majority of rhinoviruses to a susceptible cell.

10. A peptide or composition of peptides according to Claim 8, which is capable of displacing rhinovirus virions when those virions are bound to a susceptible cell.

11. A pharmaceutical composition comprising a peptide or peptides according to Claim 7 for the prevention and treatment of rhinovirus infection.

12. A formulation for intranasal instillation of a peptide or peptides according to Claim 7 for the prevention and treatment of rhinovirus infection.

13. A peptide or composition of peptides which corresponds to the Fab or Fv portion of an immunoglobulin molecule which can bind to the intercellular adhesion molecule-1, ICAM-1.

14. A peptide according to Claim 13 which prevents a ligand from binding to ICAM-1.

21

15. A peptide according to Claim 14 which binds to domain I of ICAM-1.

16. A pharmaceutical composition containing a peptide or peptides according to Claim 14.

17. A peptide or composition of peptides according to Claim 14 which can bind to and inactivate the intercellular adhesion molecule-1.

18. A pharmaceutical composition containing a peptide or peptides according to Claim 17.

FIG. IA

FIG. IB

FIG. IC

FIG. II

FIG. VI

FIG.ⅢA

pG1: HEAVY CHAIN Fd FRAGMENT

```
NdeI                                              BclI /BamHI
  4603                    5251              5266
CATATGGAGGTTCAG....AGGGATTGTGGTTAATGATCC
  METGluValGln....ArgAspCysGly
       H1                           H236
```

FIG.ⅢB

1A6 LIGHT CHAIN

5000

5279/Bgl II/BamHI

NdeI

pT7

AMP GENE

TRANSCRIPTION

1000

4000

pK1
5279

2000

3000

## FIG.IVA

pK1: LIGHT CHAIN

NdeI

4603

5236

CATATGGATATTGTG....AGGAATGAGTGTTAGAGACAAAGGTCCTGAGACGCCACCAA..

METAspIleVal....ArgAsnGluCys.

L1

L214

BglII/BamHI

5279

..CCAAGATCC

## FIG.IVB

26

pSCV1: SINGLE-CHAIN Fv

```
NdeI                                              V_L/V_H
 | 4603            4954           XhoI            4975                   5317
 | |               |              |               |                      |
 | |               |              |               |                      |
CATATGGATATTGTG..ATCTTCCCACCCTCGAGTGAGGAAGAAGTTCAGCTG..GTC..
   METAspIleVal..IlePheProProSerSerGluGluGluValGlnLeu..Val..
         |                                      |        |              |
         |                                      |        H1            H111
         L1                                     |
                                              L124
```

```
      BglII /BamHI
        5334 |
            | |
            | |
..TCTGCATAATGATCAGATCC
  SerAla
     |
   H113
```

FIG.$\underline{V}$

*FIG. VII*